# EUROPEAN PATENT APPLICATION

(11) **EP 1 804 058 A1**
(43) Date of publication of application: **04.07.2007**
(21) Application number: 05028551.9
(22) Date of filing: 28.12.2005
(51) Int. Cl.: G01N 27/453, G01N 27/447

(54) **Integrated two-dimensional gel electrophoresis**

(71) Applicant: Roche Diagnostics GmbH, 68305 Mannheim (DE); F.HOFFMANN-LA ROCHE AG, 4070 Basel (CH)
(72) Inventor: Kopp, Martin, 6332 Hagendorn (CH); Sandoz, Roger, 6343 Rotkreuz (CH); Curcio, Mario, 5643 Sins (CH); Glauser, Michael, 6343 Rotkreuz (CH)
(74) Representative: Irniger, Ernst

(57) **Abstract**

An electrophoresis assembly or disposable device for the separation of a complex protein sample, using two dimensional gel electrophoresis comprises a carrier such as a film or foil or glass plate (5) with an area for the first dimension gel strip (7) and an area (16) for the second dimension gel, where said two areas are directly in contact with each other. The assembly or device further comprises a two component main body made of a hard component (3) and a thin and flexible soft component layer (11) at a variable distance from the carrier (5) which body further comprises at least one slit or opening for external actuation of at least one valve (9, 10) where said valve is represented by said soft component material in correspondence of said at least one slit.

## Description

The present invention refers to an electrophoresis assembly or disposable device for the separation of a complex protein sample using two dimensional gel electrophoresis and a process for the separation of a complex protein sample using two dimensional gel electrophoresis.

The present invention relates to an improved disposable device and ways of processing the same, providing convenient and effective means of integrating the generally accepted and manually executed steps for the separation of a complex protein sample in proteomics analysis based on two-dimensional gel electrophoresis, thus complementing and extending the scope of the previous non published EP application 05007912.8, attached as Appendix A, in which a new method was also introduced.

### Background

Two-dimensional slab gel electrophoresis is still the most used approach to proteomics and it might be still for several years, despite alternative chromatographic methods are gaining popularity, if after improvement over the years, other limitations still present are addressed. In particular, this remains a time-consuming and laborious procedure, requiring trained personnel, on the hands of whom the quality of results is mainly depending. Just because there is much manual work involved, reproducibility is indeed difficult to achieve, whereas on the other hand gels are mostly made to be compared. Although running conditions can be quite reproducible, as these are controlled by proper set-up and power supplies, and new buffer systems have increased gel stability and performance, problems with accuracy and consistency can arise from variations in the other numerous parameters to keep under control. Some of these are for example, sample loading and rehydration, in terms of sample amount, losses, and homogeneity of the strip, strip handling with risk of damaging and contamination, imprecise and slow coupling of the strip to the gel, gel casting and polymerization, in terms of homogeneity, casting and reaction speed, especially for gradients, air sensitivity, time for completion until run is started, risk to trap bubbles causing consequently also field discontinuities, increase in temperature during the run, pH and viscosity changes, loss of buffer capacity.

Describing in details the entire process is out of the scope of this invention as several reviews can be retrieved in the literature. A brief overview is however given below to help in understanding.

Normally the first dimension separation consists of isoelectric focusing (IEF) where proteins separate according to their isoelectric point in a pH gradient, typically immobilized (IPG), in a long and narrow supported gel assuming the form and taking the name of strip. The strips, commercially available, are normally supplied in a semi-dry state and they have to be rehydrated with the sample solution before analysis. This operation takes from a few hours to typically overnight and usually takes place under mineral oil to prevent drying and crystallization of urea present in the sample solution. IEF takes place also under mineral oil for the same reasons in the same or a different tray with the strip in contact with two electrodes at the sides, between which a high voltage is applied. After IEF the strip has to be equilibrated, which means that the proteins focused within the strip have to be first alkylated and then complexed with sodium dodecyl sulfate (SDS) in order to be later transferred to the second dimension gel and separated according to size. Reduction/Alkylation can be achieved by different reagents and one has the option to perform this step during sample preparation before rehydration [1], although this might results in shifts of the isoelectric points. However SDS equilibration can be performed only after IEF, so that the strip is literally washed for several minutes in the equilibration solution containing SDS. This is then placed on top and in contact with a prepolymerized SDS polyacrylamide gel and coupling is achieved by pouring a hot agarose solution over the strip. This is usually accomplished between two glass plates which are clamped together and then placed in a buffer containing cassette where voltage is applied across the gel. The gel might be formed with a porosity gradient in order to increase resolution in the second dimension. After this is complete, the gel is removed, fixed, stained and background staining dye removed before proceeding eventually with the subsequent steps, i.e. spot picking, digesting and mass spectrometry analysis.

Ideally, what is desirable is that no further manual intervention is required after the sample has been loaded, in a way similar to the instrumental chromatographic approach, the main strength of which is indeed automation associated with better reproducibility. Automation and integration of the steps involved in the gel-based procedure is a challenge that others have already faced.

An integrated, fully automated, system mimicking step by step the manual procedure, including also sample preparation and strip casting is described in US 6554991 B1. The robotic machinery behind it, the complexity of the operation and the investment necessary go however far beyond a practical and widespread use of it, especially among the smaller research laboratories. US 2003/0127331 discloses a system where the strip once cast at the bottom of a vertical mold formed by two plates doesn't have to be moved after IEF. It is understood that the strip can be treated with the equilibration solution, apparently just from one side, and subsequently coupled to the second dimension gel by pouring the gel solution into the mold directly in contact with the strip or on top of an agarose layer. Doubts remain however concerning the efficiency and/or the time of the equilibration with the SDS having to diffuse inside the strip just from one side and whether the resolution obtained in the first dimension can be preserved. As no mention is made concerning the polymerization method, the long times associated with the classical method increase further the concern about loss of resolution. Also, the way the strip is formed and the sample is added is less reproducible and the fact that a sealing tab at the bottom of the mold has to be removed at the end is not practical. In EP 0366897 it is proposed that the strip is first separated from a prepolymerized gel by means of a non-conductive phase-change material, which is melted after IEF by increasing the temperature, removed and substituted with other gel medium. No reference is however made to the equilibration step and besides the concerns about the effect of the temperature for proteins and gel, remains the problem associated with closing and opening this time the top of the mold. Other barrier means between strip and gel are proposed in WO 02/084273 A1. The first embodiment reported therein making use of sliding solid barriers is certainly not the most advantageous as formed gels might be disrupted by this action. A more interesting solution makes use of pneumatically assisted valves consisting of soft and expandable material, separating the strip from a preferably precast gel. The space occupied by the valve is later filled with agarose for coupling. In a third embodiment, semi-walls at the sides of the strip, are used as gasket against which a foil used also as gel support can be pressed, thus opening and closing the strip chamber by changing position relative to the opposite rigid surface. The gel solution is in this case introduced and polymerized preferably after opening the strip chamber at the end of the first dimension or, with difficulty to imagine as the foil has to move, the gel can be precast. Although the possibility to immerse the strip with the gel solution in one step is claimed, the use of agarose is again preferred. Equilibration solutions can access the strip through the rigid part of the device. An automated system eventually based on this principle should be commercialized by Nextgen Sciences in the near future. There are however weak points still left in this system, first of which is represented by dead volumes for the sample regardless of the embodiment. Indeed, an extra space is necessary above the fully rehydrated strip in order to allow the flow of the equilibration solution after the first dimension, thus requiring excess of sample to fill that same gap when rehydrating the strip. Also, the fact that excess liquid is left above the strip during IEF can result in disturbed focusing and horizontal striking, and if removed can result in drying of the strip. Moreover, loss of resolution due to the long waiting time for gel polymerization and deleterious effects due to penetration of acrylamide monomers into the strip can be expected when casting the gel after IEF, as no methods of polymerizing the gel solution, other than the intended classical one, are claimed or even mentioned. This must be the reason why the use of agarose is preferred in all cases. Agarose brings however new annoying issues, like the need to be boiled before melting and the troubles to remove it from all the tubing and fittings once it has started to gel. Concerns remain also regarding the efficacy in maintaining an even and non deformed foil shape, important in guaranteeing a homogeneous gel thickness.

One object of the present invention is to propose an electrophoresis assembly or disposable device for sample separation based on two-dimensional electrophoresis, which requires only minimal or preferably no manual intervention once the sample has been loaded to the device.

It is a further object of the present invention to propose automatic operative steps of processing said device. Proposed is an electrophoresis assembly or disposable device for sample separation using two dimensional electrophoresis according to the wording of claim 1. With the present invention it shall be demonstrated that even following classical approaches as described above and still using valves within disposables for gel electrophoresis further improvements over the prior art can be achieved, providing cheaper, simpler, quicker and equally integrated solutions in the form of disposables and processing system respectively as described below and as claimed in the attached claims.

### Description of the invention

Below and with reference to the following schematic drawings a brief description of examples of systems and processes according to the present invention are given, highlighting the differences with the prior art and the advantages that can be claimed.

### System

The two-dimensional electrophoresis system comprises a disposable core part subjected to a series of automatic operative steps by an e.g. software-controlled instrument, elements of which are also part of the invention in combination with the process, the disposable format and its function. With the term instrument an arrangement comprising e.g. the buffer reservoirs, the cooling block, electrodes, tubing, UV lamps, etc. for processing the disposable is understood. The body of the electrophoresis device including at least one valve is injection molded by e.g. applying two-component molding technology, meaning that no assembly is needed between the two parts and thus reducing the cost of manufacturing. It is thus meant to be disposable and from now on it will be referred to as disposable. The material of the body is preferably chosen among PMMA, PC, PE, PET, PP or COC taking care that this is UV transparent, with the specific intent to allow UV-initiated fast polymerization of the gel solution during the process by shining light of appropriate wavelength directly through it, from one or multiple lamps integrated into the instrument. The material of the soft component for the valve is preferably chosen among TPE's (Thermoplastic elastomers) compatible with the previously chosen body material e.g. PTS - Thermoflex^{®} (Plastic Technology Service Ltd, Salisburg SP5 4BZ, UK) or Santoprene^{®} (Advanced Elestomer Systems, LP, Akron, Ohio, USA). This can also be UV transparent and is molded e.g. in a convenient flat laminar shape directly attached to the inner surface of the disposable body. By leaving two slits through the disposable body immediately flanking the zone of the strip laying underneath, the elastic soft component can be stretched down by external rigid actuation performed by the instrument thus trapping the strip in a closed and tight environment by pressing against the strip/gel carrier, e.g. a gel-bond foil or a glass plate. Similarly, slits can be designed also in other positions to divide the gel chamber in compartments if needed or simply to close any eventual open edge.

Another soft component is integrated in the disposable as a gasket rod or ring along the perimeter of the gel area and in sandwich arrangement between the inner surface of the body and the carrier foil. This could be molded at the same time and with the same procedure, eventually also with the same material as above, and exerts another important function allowing variation of the distance between the two opposite surfaces upon external applied pressure. This distance corresponds, e.g. preferably to approx 0.7 mm during rehydration and IEF and to approx 1.0 mm during equilibration, gel casting, polymerization, and second dimension separation.

The carrier foil, which cross-links to the gel during polymerization is e.g. also laminated to the disposable and can be peeled off by the user after the process.

In addition, the outer dimensions of the disposable are preferably designed according to the ANSI SBS (American National Standards Institute, The Society of Biomolecular Screening : 127.76±0.25mm x 85.48±0.23mm) standard in order to facilitate robotic handling.

One option is also to integrate cheap disposable electrodes, consisting e.g. of graphite, for the first dimension separation. In alternative electrode rods, e.g. made of platinum, going to contact the strip at the extremities through holes in the disposable body could be part of the instrument and are not disposable. Preferably integrated into the instrument are the electrodes for the second dimension, to be inserted into the buffer reservoirs of the disposable at appropriate time. Another option is to have at least one cheap electrode integrated in the disposable, e.g. at the cathode, where redox reactions and electrode consumption are less important.

In another embodiment, in order to make the disposable device even simpler and more compact, buffer reservoirs for the second dimension could also be part of the instrument clamped to the disposable when needed by simple means making use of gasket and external pressure. In order to prevent the loss of buffer capacity associated with small reservoirs and small buffer volumes, the buffer could be freshly circulated from larger reservoirs upstream. Keeping the entire circuit under the applied voltage could be avoided in several ways, e.g. using a "stop and go" discontinuous approach opening the circuit at intervals to replace the buffer, or restricting the channel of communication between large and small reservoirs, eventually also dispensing air bubbles along the liquid path as insulators. Alternatively the limitation of the buffer capacity can be overcome by recirculating the cathode buffer with the anode buffer and vice versa. By this means the buffer reservoirs can be kept small with no additional need of buffer during the run.

In order to maintain the flatness and evenness of the carrier foil, important especially to guarantee gel homogeneity and efficient cooling, both fundamental for reproducibility, the cooling block of the instrument on which the disposable is geometrically fitting can be made of porous ceramics, metal or other heat-conductive alloy, through which vacuum suction can be applied. At the same time this represents an advantageous way to steadily fix the disposable into the instrument so that it could be even rotated 90° during gel casting by mechanical rotation of the cooling block.

The disposable can be generic containing no IEF strip, thus leaving the freedom to the operator to insert the desired strip with the desired pH range, and avoiding the need to deliver and store the entire disposable with the strip inside at refrigeration temperature. Guiding features are provided so that no misplacing can occur, e.g. with closed valves, while the electrodes could serve also to keep the strip in place. It is however possible, especially if the disposable is made even simpler and compact as suggested above, that the strip is already integrated into the disposable, so that one could order different sets of disposables containing different strips. Another possibility is to have the strip already attached on the foil with the foil being delivered separately from the disposable body. In this case, the user has to assemble then the two parts together, e.g. with the help of positioning holes. One other option is to bring in the strip through an opening in the cover foil which will be closed afterwards with a tape-like mechanism.

Another element applicable to embodiments with valves is the use of membranes or blade at the gel/buffer interface. What is claimed is the use of hydrophobic, eventually supported, membranes with the right combination of material, thickness and pore size, made e.g. from PET, PE, PP or PES ,with the function of acting as a barrier for the liquid gel solution during even vertical casting but allowing liquid and normal electrical contact between polymerized gel and SDS-containing buffer. A working example is the Ultran^{®} PES (Polyethersulfone) 5 KD membrane from Schleicher & Schuell, Einbeck, DE, (Watman), normally used for filtration and biological applications, but others can be employed as well, more or less efficiently depending also on the fact whether the gel contains SDS or not. In order to keep the disposable as simple as possible, one could also imagine that slits at the gel/buffer interface are created only after gel polymerization in the form of longitudinal cut along a thinner integral lining of the moulded disposable body or cutting the soft-component material through slits in the hard component body by means of blades integrated into the instrument. Perhaps a simpler solution, when using anyway valves to enclose the strip is by means of the soft component, injection-molded together with the disposable body, closing the open edges of the gel chamber during casting and polymerization, upon external actuation, at the same way as for closing the strip chamber. Another simple solution is to have a tape across the slit which can be removed as soon as the contact to the buffer has to be established. The simplest of all solutions is to cast the gel with the strip at the bottom leaving only the external valve at the side of the strip closed.

Below and with reference to the following schematic drawings a brief description of examples of systems and processing according to the present invention are given, highlighting the differences with the prior art and the advantages that can be claimed.

In the Drawings:
Figures 1a and 1b show schematically and not to scale the two-component disposable core part with closed valves and two variable thicknesses, one during rehydration and IEF and the second thickness during equilibration respectively,
Fig. 2a and 2b show schematically and not to scale two possible embodiments for the two component disposable core part during gel-casting in the second dimension
Fig. 3 refers to the slits at the gel/buffer interface sealed by appropriate membrane or tape,
Fig. 4 refers to a possible way to create slits at the gel/buffer-interface by means of a blade function integrated in the instrument, only after gel-casting and polymerisation, and
Fig. 5 shows schematically one possible embodiment in which the buffer reservoirs for the second dimension are not integrated in the disposable but are clamped by the instruments when needed, while buffer is freshly circulated from larger reservoirs upstream in order to prevent the loss of buffer capacity associated with small reservoirs and small buffer volumes.

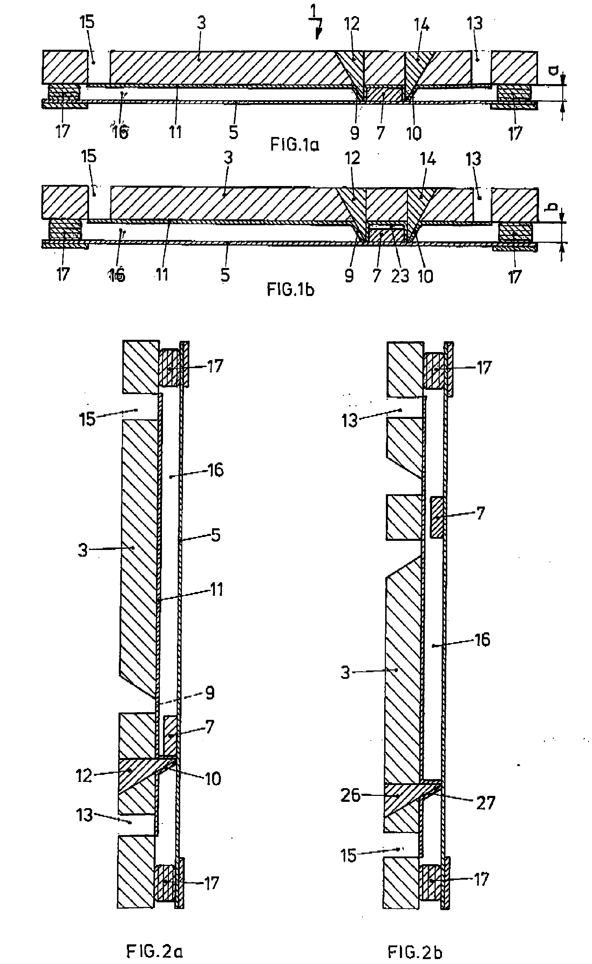

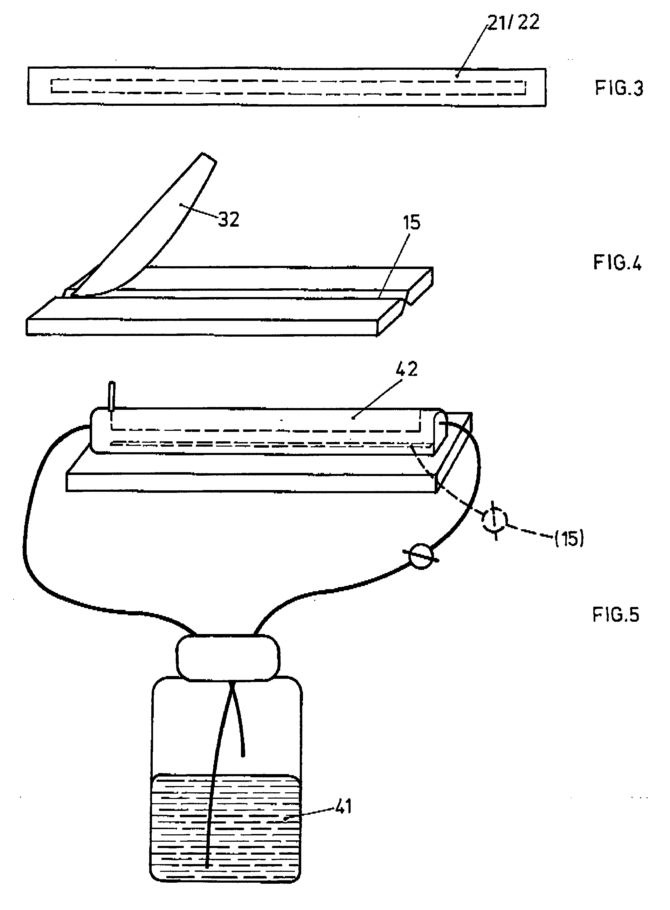

The process steps, automatically executed and described with reference to the attached figures are the following:
1. Insert the first gel strip 7 or assemble the foil 5 carrying the strip 7 into the disposable 1 unless the strip is already integrated in the closed disposable 1. A possible alternative is also that to cast the IEF strip 7 in situ with the two valves 9 and 10 arranged on both sides closed. The valves may be actuated by inserts 12 and 14, being part of the instrument (not shown) provided to process the disposable 1.
2. Load the sample in rehydration solution with the two valves 9 and 10 at both sides of the strip 7 closed and with a distance of e.g. 0.7 mm between the foil 5 and the soft-component valve material 11 covering the inner surface of the disposable body 3 until filling the strip chamber thus created.
3. Wait for rehydration at least e.g. one hour having set the temperature of the cooling block (not shown) at e.g. 30° - 35° Celsius.
4. Run IEF applying ramping high voltage between the two electrodes (not shown) either integrated or inserted at this moment by the processing instrument, which controls also the temperature e.g. set at 20° Celsius.
5. Increase the distance a as shown in fig. 1a from e.g. 0.7 mm between foil 5 and inner surface 11 to distance b, e.g. 1.0 mm as shown in fig. 1b, by releasing the instrument applied pressure from the device while stretching further the soft-component valves 9 and 10 with the aid of the external inserts 12 and 14 to maintain the chamber of the strip 7 closed. The increase of the distance a according fig. 1a to b according to fig. 1b is achieved as the integrated gasket 17, which is made e.g. out of an elastic soft component material, expands returning to its original shape. In the arrangement as shown in fig. 1a the disposable body 3 is forced downwards towards the foil 5 by applying an external pressure, which is removed in the arrangement according to fig. 1b.
6. Flow the alkylation / SDS equilibration solutions into the e.g. 0.3 mm deep channel 23 created on top of the strip 7 and empty at last.
7. Rotate the whole disposable 1 by 90° to bring it in vertical position as shown e.g. in figures 2a and 2b. This movement can be achieved upon rotation of the cooling block (not shown) to which the disposable is steadily fixed by geometrical fitting and vacuum suction through the porous material as proposed above.
   Fig. 2a shows the design of the disposable as shown in fig. 1b in vertical position with valve 10 on one side of the strips 7 still in closed position and valve 9 being opened.
8. Unless the slits 13 and 15 at the gel buffer interface have not yet been created or if they are closed by either the soft component layer 11 or the additional membranes 21 and 22 as shown in fig. 3, the valve 10 according to the embodiment of figure 2a with strip 7 at the bottom has to remain closed until the gel solution is cast and polymerized. As an alternative according to fig. 2b with the gel strip 7 at the top an additional external valve 27 is arranged at the opposite side of the disposable 1. (Spacing between foil 5 and cover plate 3 still e.g. 1 mm which means according to distance b of fig. 1b).
9. Cast the gel solution for forming the gel of the second dimension separation through e.g. a hole (not shown) in the disposable body 3 thus achieving at the same time coupling with the strip 7, and polymerise by fast UV initiated reaction completed e.g. in less than 5 minutes which is possible due to the UV transparent disposable body 3 and layer 11. If desired also gradient gels can be cast.
10. Open valve 10 within the arrangement of fig. 2a or valve 27 activated by insert 26 within the arrangement of fig. 2b, so that the running buffer can contact the gel for the second dimension separation, or in alternative create the slits 13 and/or 15 if these were not yet present by e.g. a blade function 32 as shown in fig. 4. Another alternative is to proceed soon to the next step if the slits 13 and 15 were closed by additional membranes 21 and 22 as shown in fig. 3.
11. Eventually rotate back the arrangement to horizontal position if open buffer reservoirs are used, otherwise back rotation is not absolutely necessary. If the reservoirs are part of the instrument, which means not integrated into the disposable they can now be joined as shown with reference to fig. 5 and designated wit the reference numbers 41 and 42.
12. Introduce the running buffer, eventually circulate the running buffer and initiate the second dimension run at controlled temperature as e.g. 20° Celsius, by applying voltage between the two reservoirs, the electrodes being either integrated into the disposable or in the instrument.
13. Remove after the second dimension run is completed the disposable 1 from the instrument and open it to remove the gel by pealing off the foil 5, to which the gel is bonded.

The above described process with reference to the attached drawings is of course an example suitable for describing the present invention and is not at all limiting the present invention. The type of material used for producing the disposable, including soft component for the valves, the foil, compressible parts, etc. could be changed in an appropriate manner. The use of a UV or light transparent material for the disposable is preferred so that UV or light initiated polymerisation of the second gel is possible, but it should not be a limiting factor to the present invention. Furthermore using two, three or more valves is possible. One important feature of the present invention of course is, that the distance between the inner disposable body surface and the foil, on which the first gel strip is attached is variable, which means that after the first dimension separation the distance can be expanded e.g. due to the arranged elastic gasket.

The combination of a valve around the strip with the variable distance between the cover foil and the inner disposable surface (e.g. 0.7 mm and 1.0 mm during first and second dimension respectively) raises the number of allowed positions for the valve from two to three and offers important advantages compared to the prior art. First of all, no or minimum sample excess is required to rehydrate the strip as the volume of the strip chamber created by the valves corresponds to the volume of the rehydrated strip. In this way, also IEF can be run under optimal conditions with no liquid excess on top of the strip. Space is created on top of the strip only after IEF to introduce flowing equilibration solutions, thus providing also optimal equilibration conditions. Finally, as a thicker gel with a small space above the strip is required to achieve proper coupling and perform a good second dimension analysis in terms of field homogeneity, 2D resolution and reproducibility, optimal conditions are provided also in this subsequent step.

Unlike the prior art, UV-initiated fast polymerization is adopted in the field of two-dimensional gel electrophoresis, choosing an intiator that is stable in the acrylamide gel solution until exposed to a light source whose wavelength range comprises its abosorbance spectrum. Valves are used only to close the strip in a tight environment and not as barriers between the gel and the strip because the gel can be polymerized quickly after the first dimension and doesn't have to be precast. Thus chemistry, storage time and conditions as well as waiting time for post- or pre- IEF polymerization are no longer an issue. Just because polymerization proceeds fast, as disclosed in the prior non published EP-Application 05007912.8, which is attached as Appendix A, the use of a coupling gel like e.g. agarose can also be eliminated. The gel solution can now fill completely the mold, contacting, covering and enclosing the strip, while this is not possible with the traditional method, making use of ammonium persulfate (APS) and N,N,N',N'-tetramethylethylenediamine (TEMED) as initiator and catalyst respectively of radical polymerization. These reagents indeed have to be added and mixed at the last moment as they start immediately polymerization already during casting, thus causing already preparation problems, and because the reaction proceeds slowly taking normally more than one hour to be completed, loss of resolution obtained during the first dimension and diffusion of acrylamide monomers into the strip, causing possible crosslinking with the proteins, become other important issues.

For the same reasons, fast UV polymerization becomes also particularly convenient when casting gradient gels.

### References

[1] Herbert et al. Electrophoresis 2001, 22, 2046-2057.

## Claims

1. Electrophoresis assembly or disposable device for the separation of a complex protein sample using two-dimensional gel electrophoresis comprising,
a carrier such as a film or foil or glass plate (5) with an area for the first dimension gel strip (7) and an area (16) for the second dimension gel where said two areas are directly in contact with each other,
a two-component main body made of a hard component (3) and a thin and flexible soft-component layer (11) at variable distance from the carrier (5) further comprising at least one slit or opening for external actuation of at least one valve where said valve is represented by said soft-component material in correspondence of said slit.

2. Assembly or disposable device according to claim 1 **characterised in that**, the main disposable body is at least partially UV transparent.

3. Assembly or disposable device respectively according to one of the claims 1 or 2 **characterised in that** the soft-component layer (11) located at the inner surface of the disposable body can be stretched down towards the gel carrier (5) upon external actuation through the at least one slit in the hard component (3) in correspondence of said slit.

4. Assembly or disposable device according to one of the claims 1 to 3 **characterised in that**, the hard component (3) comprises at least two slits or openings for external actuation of the soft component layer acting as valves (9, 10) on both sides of the area of the first dimension gel strip (7) and/or as valves for the second dimension gel at appropriate positions.

5. Assembly or disposable device according to one of the claims 1 - 4 **characterised in that**, the variation of distance between the main disposable body and the gel carrier is obtained by means of at least one elastic compressible/expandable sealing or gasket in sandwich arrangement between said main disposable body and carrier, preferably along the perimeter of the second dimension gel area and being part of said disposable device.

6. Assembly or disposable device according to one of the claims 1 to 5 **characterised in that**, the hard component (3) further comprises holes or connecting fitting for sample loading, flowing of the equilibration solutions, casting of the gel solution or venting.

7. Assembly or.disposable device according to one of the claims 1-6 **characterised in that**, the hard component (3) is preferably chosen among PMMA, PC, PE, PET, PP or COC (Cycloolefin-Copolymer).

8. Assembly or disposable device according to one of the claims 1-7 **characterised in that**, the soft-component (11) is preferably chosen among thermoplastic elastomers like e.g. PTS - Thermoflex^{®} or Santoprene^{®} compatible with the material of the hard component to enable two-component injection moulding and such that the second dimension gel does not stick to it.

9. Assembly or disposable device according to one of the claims 1 to 8 **characterised in that**, the disposable (1) further comprises electrodes for the first and/or second dimension and/or arrangement for external introduction.

10. Assembly or disposable device according to one of the claims 1 to 9 **characterised in that**, the main disposable body further comprises slits at the interface between second dimension gel and running buffer.

11. Assembly or disposable device according to claim 10 **characterised in that**, the slits at the interface between second dimension gel and running buffer are sealed by additional membranes (21, 22).

12. Assembly or disposable device according to claim 11 **characterised in that**, the additional membranes (21, 22) have the right combination of thickness, pore size and material, with the function of acting as a barrier for the liquid gel solution during casting but allowing liquid and normal electrical contact between polymerized gel and SDS-containing buffer. (Such as e.g. PET, PE, PP or PES)

13. Assembly or disposable device according to claim 11 or 12 **characterised in that**, the membrane is PES (Polyethersulfone) such as e.g. Ultran^{®} 5 KD.

14. Assembly or disposable device according to claim 10 **characterised in that**, the slits at the interface between second dimension gel and running buffer are being created only after gel polymerization by means of a blade function.

15. Assembly or disposable device according to one of the claims 1-14 **characterised in that**, the outer dimensions of said disposable device are preferably designed according to the ANSI SBS (American National Standards Institute, The Society of Biomolecular Screening: 127.76±0.25mm x 85.48±0.25mm) standard in order to facilitate robotic handling.

16. Assembly according to one of the claims 9 - 15 comprising at least one large external buffer reservoir from which running buffer for the second dimension separation can be freshly supplied to and/ or recirculated from smaller buffer reservoirs either integrated with or external to the disposable device in correspondence of holes, slits or openings according to claim 9 or 10.

17. Assembly according to one of the claims 1 - 16 **characterized in that**, the disposable device is geometrically fitting and preferably steadily fixed with the external carrier side on a cooling block made e.g. of a porous material such as ceramics, metal or other heat-conductive alloy, through which vacuum suction can be applied.

18. Process for the separation of a complex protein sample using two-dimensional gel electrophoresis within an assembly or disposable device according to one of the claims 1 - 17 **characterised in that**, the sample is loaded in a rehydration solution onto a first dimension gel strip arranged on a carrier with valves closed on both sides of the strip with the distance between the carrier and the main disposable body corresponding approximately to the thickness of the rehydrated strip and **in that**, after rehydration and separation in the first dimension, the distance between the carrier and the main disposable body is increased while stretching further down the valves at both sides of the strip to maintain the strip environment closed and thus creating a free space or channel above the strip to allow flowing of the equilibration solutions.

19. Process according to claim 18, **characterised in that**, a gel solution for the second dimension separation gel is introduced with at least one valve at one side of the strip open and the increased distance between the carrier and the main disposable body thus achieving coupling with the strip at the same time followed by UV initiated polymerization.

20. Process according to one of the claims 18 or 19, **characterised in that**, casting of the gel solution of the second dimension gel are executed by having the disposable device in a more or less vertical position.

21. Process according to one of the claims 18 - 20, **characterised in that**, for performing successive analyses of the separated sample, the carrier, preferably a foil or film with gel-bond properties, is peeled off from the disposable together with the attached gel.
